# FASCICULE DE BREVET EUROPEEN

(11) **EP 1 389 120 B1**
(45) Date de publication et mention de la délivrance du brevet: **01.08.2012**
(21) Numéro de dépôt: 02732883.0
(22) Date de dépôt: 23.05.2002
(51) Int. Cl.: A61K 31/415, A61K 31/4174, A61K 31/4196, A61K 31/496, A61K 31/497, A61K 31/513, A61K 31/7008, A61K 31/7012, A61K 31/7048, A61P 11/06, A61P 11/14, A61P 17/02, A61P 17/06, A61P 19/02, A61P 31/10, G01N 33/569

(54) **Utilisation de la nystatine pour le traitement de l'athérosclérose liée à la présence de champignons parasites**
Verwendung von Nystatin zur Behandlung von Atherosklerose, welche mit der Präsenz von parasitären Pilzen einhergeht
Use of nystatin for the treatment of atherosclerosis linked to the presence of parasitic fungi

(30) Priorité: 23.05.2001 FR 0106775; 11.07.2001 FR 0109203
(43) Date de publication de la demande: 18.02.2004
(73) Titulaire: Antonov, Roumen, 75006 Paris (FR)
(72) Inventeur: Antonov, Roumen, 75006 Paris (FR)
(74) Mandataire: Grosset-Fournier, Chantal Catherine
(86) Numéro de dépôt international: PCT/FR2002/001739
(87) Numéro de publication internationale: WO 2002/094287

(56) Documents cités:
- WO-A-00/24402
- WO-A-00/24403
- FR-A- 2 644 476
- US-A- 4 491 588
- US-A- 5 358 959
- US-A- 5 880 101
- NICZYPORUK W ET AL: "EFFECT OF TOPICAL MICONAZOLE IN PLAQUE PSORIASIS" ROCZNIKI AKADEMII MEDYCZNEJ W BIAOYMSTOKU, MEDICAL ACADEMY OF BIAYSTOK, BIAYSTOK,, PL, vol. 42, no. 1, 1997, pages 236-240, XP008000924 ISSN: 0067-6489
- KOLLMANN M ET AL: "2%IGE KETOCONAZOL-WASCHLOESUNG (TERZOLIN ) IN DER BEHANDLUNG DES LEICHTEN UND MITTELSCHWEREN ATOPISCHEN EKZEMS - UNTERSUCHUNGEN ZUR WIRKSAMKEIT UND VERTRAEGLICHKEIT" H + G. ZEITSCHRIFT FUER HAUTKRANKHEITEN, GROSS VERLAG, BERLIN, DE, vol. 72, no. 11, 1997, pages 812-815, XP008000905 ISSN: 0301-0481
- ROEGEL E ET AL: "ASPERGILLOSES INVASIVES ET EMBOLLE OU INFARCTUS PULMONAIRES. A PROPOS DE 5 OBSERVATIONS" REVUE DES MALADIES RESPIRATOIRES, PARIS, FR, vol. 2, no. 3, juin 1985 (1985-06), pages 182-183, XP008000866 ISSN: 0761-8425
- ROEGEL E ET AL: "INVASIVE ASPERGILLOSIS AND EMBOLISM OR PULMONARY INFARCTION REPORT OF 5 CASES" REVUE DES MALADIES RESPIRATOIRES, vol. 2, no. 3, 1985, pages 182-183, XP008000866 ISSN: 0761-8425
- LELCUK S ET AL: "THROMBOXANE A-2 MODERATES PERMEABILITY AFTER LIMB ISCHEMIA" ANNALS OF SURGERY, vol. 202, no. 5, 1985, pages 642-646, XP008000865 ISSN: 0003-4932
- NAUMANN RALPH ET AL: "Cunninghamella bertholletiae infection mimicking myocardial infarction." CLINICAL INFECTIOUS DISEASES, vol. 29, no. 6, décembre 1999 (1999-12), pages 1580-1581, XP008000869 ISSN: 1058-4838
- CHERA E ET AL: "EFFECTS OF DIFFERENT DOSES OF HYPOCHOLESTEROLEMIC POLYENES ON THE SERUM IONS" REVUE ROUMAINE DE BIOLOGIE SERIE DE BIOLOGIE ANIMALE, vol. 30, no. 2, 1985, pages 101-108, XP001061281 ISSN: 0035-3922
- STEINER, ALFRED ET AL: "Effect of antibiotics on serum cholesterol concentration of patients with atherosclerosis" CIRCULATION (1961), 24, 729-35, XP008000893

## Description

La présente invention a pour objet la mise en évidence de champignons parasites dans l'organisme humain, et l'utilisation d'antifongiques, tels que ceux actifs sur les champignons du genre *Candida,* pour la préparation de médicaments destinés à la prévention ou au traitement de pathologies, chez l'homme ou l'animal, liées à la présence dans l'organisme humain ou animal de ces champignons.

Des expériences récentes menées à partir d'échantillons de vaisseaux humains comprenant le cas échéant des plaques d'athérome, ont permis à l'Inventeur de conclure sans ambiguïté sur la présence d'au moins un champignon choisi parmi *Entomocorticum sp., Stereum annosum, Phellinus igniarus, Peniophora pithya, Peniophora piceae, Xi*/*aria longipes, Rosellinia arcuata, Rosellinia necatrix, Mulriclavula vernalis, Hericium coralloides, Hericium abietis, Hericium erinaceum, Hericium rivularis, Tricholoma lerreum,* dans l'organisme humain, notamment dans les plaques d'athérome, et autres tissus sclérotiques, à savoir de champignons d'espèces voisines ayant un biotope commun, et plus particulièrement une activité biologique commune, à savoir la dégradation du bois.

Chera E. et al. (Rev. Roum. Biol. Biol.Anim., 30, no 2, p. 101- 107) décrivent l'effet de la 7-nystatine et de la CM-nystatine sur les taux sériques des ions calcium, potassium et sodium chez des lapins soumis à un régime hypocholestérolémiants. Les résultats obtenus montrent que ces composés restaurent les taux normaux de ces ions confirmant que la nystatine et ses dérivés ont bien des propriétés hypocholestérolémiantes.

Les demandes internationales WO 02/24402 et WO 02/24403 décrivent l'utilisation d'antifongiques actifs sur les champignons du genre *Candida* et éventuellement actifs sur les champignons du genre *Rodhotorula* dans le traitement de certaines maladies liées à ces champignons, notamment l'athéroscléroses. Les champignons mentionnés sont différents de ceux de ma présente invention.

L'invention a pour objet l'utilisation de la nystaline en tant qu'antifongique pour la préparation d'un médicament destiné à la prévention ou au traitement de l'athérosclérose liée à la présence dans les vaisseaux sanguins de l'organisme humain ou animal d'au moins un champignon choisi parmi *Stereum annosum* et/ou *Phellinus igniarus. et*/*ou* Entomocorticum s.p.

L'invention concerne plus particulièrement l'utilisation de la nystatine, en tant qu'antifongique pour L'invention concerne plus particulièrement l'utilisation de la Nystatine, pour la préparation d'un médicament destiné à la prévention ou au traitement de l'athérosclérose liée à la présence dans l'organisme humain ou animal, des champignons filamenteux *Phellinus igniarus* déposés le 3 juillet 2001 à la Collection Nationale de Cultures de Microorganismes (CNCM) de L'institut Pasteur sous le numéro I-2690, et/ou des champignons filamenteux *Stereum annosum* déposés le 3 juillet 2001 à la CNCM sous le numéro I-2691, et/ou des champignons filamenteux *Entomocorticum sp.* déposés le 3 juillet 2001 à la CNCM sous le numéro I-2692.

La nystatine répond à la formule la

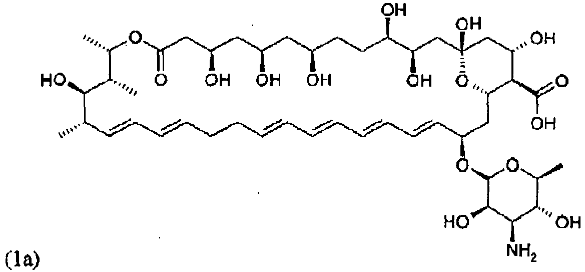

Avantageusement, les doses de Nystatine utilisées dans le cadre de la présente invention sont environ 2 à environ 100 fois, notamment environ 5 à environ 50 fois, inférieures aux doses usuelles (à savoir aux doses usuelles d'environ 10 comprimés de 500 000 UI par jour pendant 10 jours) dans le cadre du traitement des candidoses notamment buccopharyngées, intestinales ou vaginales.

De plus, la durée du traitement est beaucoup plus longue, notamment au moins environ 5 fois plus longue, dans le cas de la présente invention, que dans le cas du traitement des candidoses susmentionnées, et est plus particulièrement d'au moins environ 2 mois, notamment d'au moins environ 6 mois.

A titre d'illustration, les posologies utilisées dans le cas de l'utilisation de la Nystatine, dans le cadre de la présente invention, sont les suivantes :
- moins d'environ 100.000 unités par jour chez l'adulte, notamment chez les personnes ayant entre environ 20 ans et environ 30 à 40 ans, ou
- entre environ 25.000 et environ 50.000 unités par jour chez les personnes de moins de 20 ans environ, et chez les enfants,
- entre environ 25 000 et environ 125 000 unités par semaine, avantageusement environ 25.000 unités par semaine, chez les personnes de plus de 40 ans, pendant une durée de traitement illimitée.

Les posologies indiquées ci-dessus, sont données pour une ou plusieurs prises quotidiennes pendant la durée du traitement, ou, de préférence, pour une ou plusieurs prises par jour espacées par des intervalles d'environ 3 à environ 7 jours pendant la durée du traitement.

Aux posologies indiquées ci-dessus, la durée du traitement est d'environ au moins 6 mois pour les enfants et les personnes de moins de 20 ans environ, jusqu'à une ou plusieurs années chez les personnes de plus de 20 ans.

On peut noter qu'après de telles périodes de traitement, le système immunitaire devient capable de prendre le relais contre le développement de ces scléroses ou nécroses dues au développement de champignons tels que décrits ci-dessus, par un processus d'immunisation, et ce pendant une période comprise entre environ 3 ans et environ 5 ans. Le traitement par l' antifongique susmentionné peut alors cesser.

Dans le cas où cette immunisation viendrait à disparaître, le traitement à l'aide de la Nystatine peut alors reprendre, de préférence avec des posologies environ deux fois inférieures à celles indiquées ci-dessus, et pendant une durée environ deux fois plus courte.

Les posologies et durées de traitement indiquées ci-dessus permettent de diminuer de façon significative les quantités de champignons tels que décrits ci-dessus dans l'organisme.

Avantageusement, la Nystatine est utilisée, dans le cadre de la présente invention, pour la préparation de médicaments susceptibles d'être administrés par voie orale, notamment sous forme de comprimés ou de suspensions buvables, ou par voie injectable.

L'invention est illustrée à l'aide du tableau I ci-après qui représente les pourcentages en nombre de toutes les espèces isolées à partir de biopsies de vaisseaux sanguins de patients présentant, le cas échéant, des plaques d'athérome, lesdites espèces ayant été identifiées par séquençage.

**TABLEAU 1**

| ESPECES | Pourcentage en nombre de toutes les espèces isolées |
|---|---|
| *Entomocorticium sp.* | 28,5% |
| *Siereum annosum* | 28,5% |
| *Phellinus ignimmus* | 14% |
| *Peniophora pithya,* or *P. piceae,* | 7% |
| *Xilaria longipes,* or *Rosellinia arcuata,* or *R necatrix,* | 7% |
| *Multiclavula vernalis,* or *Hericium coralloides,* or *H. abietis, or H. erinaceum,* or *H. rivularis, or Tricholoma terreum,* | 14% |

## Revendications

1. Utilisation de la nystatine en tant qu'antifongique pour la préparation d'un médicament destiné à la prévention ou au traitement de l'athérosclérose liée à la présence dans les vaisseaux sanguins de l'organisme humain ou animal, d'au moins un champignon choisi parmi *Stereum annosum* et/ou *Phellinus igniarus et*/*ou Entomocorticum sp.,*

2. Utilisation selon la revendication 1 **caractérisée en ce que** le champignon est choisi dans le groupe comprenant *Phellinus igniarus* déposés à la Collection Nationale de Cultures de Microorganismes (CNCM) de L'Institut Pasteur sous le numéro 1-2690, et/ou des champignons filamenteux *Stereum annosum* déposés à la CNCM sous le numéro 1-2691, et/ou des champignons filamenteux *Entomocorticum sp.,* déposés à la CNCM sous le numéro 1-2692.

3. Utilisation selon l'une des revendications 1 ou 2, **caractérisée en ce que** la Nystatine est utilisée aux posologies suivantes :
- moins d'environ 100 000 unités par jour chez l'adulte, notamment chez les personnes ayant entre environ 20 ans et environ 30 à 40 ans, ou
- entre environ 25 000 et environ 50 000 unités par jour chez les personnes de moins de 20 ans environ, et chez les enfants, ou
- entre environ 25 000 et environ 125 000 unités par semaine, avantageusement environ 25.000 unités par semaine, chez les personnes de plus de 40 ans, pendant une durée de traitement illimitée,

4. Utilisation selon la revendication 3, à raison d'une ou plusieurs prises quotidiennes pendant la durée du traitement, ou, de préférence, à raison d'une ou plusieurs prises par jour espacées par des intervalles d'environ 3 à environ 7 jours pendant la durée du traitement, la durée du traitement étant d'environ au moins 6 mois pour les enfants et les personnes de moins de 20 ans environ, jusqu'à une ou plusieurs années chez les personnes de plus de 20 ans.

## Claims

1. Use of Nystatine as an antifungal for the preparation of a medicament intended for the prevention or treatment of atherosclerosis linked to the presence in the blood vessels of the human or animal organism of at least one fungus chosen from *Stereum annosum and*/*or Phellinus igniarus and*/*or Entomocorticum sp..*

2. Use according to claim 1, **characterised in that** the fungus is selected from the group comprising *Phellinus igniarus* deposited at the "Collection Nationale de Cultures de Microorganismes" (CNCM) of Pasteur Institute ("Institut Pasteur") under the number 1-2690, and/or of the filamentous fungi *Stereum annosum* deposited at the CNCM under number 1-2691, and/or of filamentous fungi *Entomocorticum sp.* deposited at the CNCM under number 1-2692.

3. Use according to one of claims 1 or 2, **characterised in that** Nystatine is used at the following doses:
- less than approximately 100,000 units per day in adults, in particular in persons of between approximately 20 years and approximately 30 to 40 years of age, or
- between approximately 25,000 and approximately 50,000 units per day in persons of less than approximately 20 years of age, and in children, or
- between approximately 25,000 and approximately 125,000 units per week, advantageously approximately 25,000 units per week, in persons of more than 40 years of age, for an unlimited treatment period.

4. Use according to claim 3, at the rate of one or more daily doses for the duration of the treatment, or, preferably, at the rate of one or more doses per day spaced out at intervals of approximately 3 to approximately 7 days for the duration of the treatment, the duration of the treatment being from approximately at least 6 months for children and persons of less than approximately 20 years of age; up to one or more years in persons of more than 20 years of age.

## Patentansprüche

1. Verwendung von Nystatin als Antimykotikum zur Herstellung eines Arzneimittels zur Vorbeugung oder zur Behandlung von Arteriosklerose in Verbindung mit dem Vorkommen mindestens eines Pilzes ausgewählt aus *Stereum annosum* und/oder *Phellinus igniarus* und/oder *Entomocorticum sp.* in den Blutgefäßen eines menschlichen oder tierischen Organismus.

2. Verwendung nach Anspruch 1, **dadurch gekennzeichnet, dass** der Pilz ausgewählt ist aus der Gruppe umfassend *Phellinus igniarus,* hinterlegt bei der Collection Nationale de Cultures de Microorganismes (CNCM) des Institut Pasteur unter der Nummer 1-2690 und/oder den filamentösen Pilzen *Stereum annosum,* hinterlegt bei der CNCM unter der Nummer 1-2691 und/oder den filamentösen Pilzen *Entomocorticum sp.,* hinterlegt bei der CNCM unter der Nummer 1-2692.

3. Verwendung nach einem der Ansprüche 1 oder 2, **dadurch gekennzeichnet, dass** das Nystatin in den folgenden Dosierungen verwendet wird:
- weniger als etwa 100 000 Einheiten pro Tag bei Erwachsenen, insbesondere bei Personen, die zwischen ungefähr 20 Jahre und ungefähr 30 bis 40 Jahre alt sind oder
- zwischen etwa 25 000 und etwa 50 000 Einheiten pro Tag bei Personen, die weniger als 20 Jahre alt sind und bei Kindern oder
- zwischen etwa 25 000 und etwa 125 000 Einheiten pro Woche, vorteilhafterweise etwa 25 000 Einheiten pro Woche bei Personen, die über 40 Jahre alt sind, während einer unbegrenzten Dauer der Behandlung.

4. Verwendung nach Anspruch 3 für eine oder mehrere tägliche Einnahmen während der Dauer der Behandlung oder vorzugsweise für eine oder mehrere Einnahmen pro Tag verteilt über Intervalle von etwa 3 bis etwa 7 Tagen während der Dauer der Behandlung, wobei die Dauer der Behandlung etwa mindestens 6 Monate für Kinder und Personen, die unter 20 Jahre alt sind, bis zu einem oder mehreren Jahren bei Personen, die über 20 Jahre alt sind, beträgt.
